# DEMANDE DE BREVET EUROPEEN

(11) **EP 1 340 452 A1**
(43) Date de publication de la demande: **03.09.2003**
(21) Numéro de dépôt: 03290404.7
(22) Date de dépôt: 19.02.2003
(51) Int. Cl.: A61B 5/00

(54) **Système d'analyse optique d'un tissu vivant**

(30) Priorité: 28.02.2002 FR 0202509
(71) Demandeur: COMPAGNIE INDUSTRIELLE DES LASERS CILAS, F-91460 Marcoussis (FR)
(72) Inventeur: De Witte, Olivier, 91190 Gif-sur-Yvette (FR)
(74) Mandataire: Bonnetat, Christian

(57) **Abrégé**

- Système d'analyse optique d'un tissu vivant.
- Le système (1) comporte un ensemble (E) de n dispositifs d'analyse optique, n étant supérieur ou égal à 3, dont chacun met en oeuvre une technique d'analyse particulière du tissu vivant (Tv) de manière à pouvoir émettre une information positive ou négative sur la présence d'une pathologie particulière, des moyens de sélection (2) actionnables, pour sélectionner un nombre p desdits n dispositifs, p étant supérieur à 1 et inférieur à n, des moyens de déclenchement (3) actionnables, pour déclencher chacun des p dispositifs qui ont été sélectionnés, et une unité centrale (4) qui, si au moins q desdits p dispositifs qui ont été sélectionnés et déclenchés, ont tous émis une information négative, q étant supérieur à 1 et inférieur ou égal à p, émet un diagnostic négatif sur la présence de ladite pathologie sur le tissu vivant (Tv).

## Description

La présente invention concerne un système d'analyse optique d'un tissu vivant.

Bien que non exclusivement, ce système d'analyse optique est destiné plus particulièrement à rechercher par voie optique une pathologie spécifique, par exemple un cancer, sur un tissu humain, c'est-à-dire sur un organe du corps humain tel qu'un poumon ou le foie par exemple.

A cet effet, il est bien connu, dans le domaine du diagnostic médical par voie optique, d'illuminer un tissu vivant humain à analyser par un faisceau lumineux de faible puissance en provenance soit de sources lumineuses usuelles, soit de sources laser, et d'effectuer, par l'analyse d'un rayonnement lumineux induit qui est ré-émis par le tissu vivant ainsi illuminé, une mesure physique caractéristique d'un paramètre physiologique particulier relatif à la pathologie recherchée de manière à pouvoir en déduire la présence ou non de cette pathologie sur ce tissu humain.

Généralement, un dispositif destiné à réaliser une telle analyse optique comporte, de façon connue :
- au moins une source lumineuse susceptible d'engendrer au moins un faisceau lumineux ;
- au moins une première fibre optique (d'émission) susceptible de transmettre un faisceau lumineux engendré par ladite source lumineuse sur le tissu vivant à analyser ;
- au moins une seconde fibre optique (de réception) susceptible de recevoir et de transmettre un rayonnement lumineux provenant dudit tissu vivant illuminé par ledit faisceau lumineux ;
- au moins un capteur optique qui est susceptible de mesurer un paramètre particulier dudit rayonnement lumineux transmis par ladite seconde fibre optique ; et
- un moyen de traitement pour émettre, au moins en fonction des mesures réalisées par ledit capteur optique, une information positive ou négative sur la présence d'une pathologie particulière sur ledit tissu vivant analysé.

Une telle analyse par voie optique, pour laquelle il suffit généralement d'amener au contact ou à proximité du tissu à analyser lesdites fibres optiques qui ont en général un diamètre d'environ un millimètre, présente, en raison de sa très faible invasivité, un avantage important, à savoir une grande tolérance de la part des patients, par rapport notamment aux biopsies qui sont généralement réalisées sur les tissus vivants. Un autre avantage d'une analyse par voie optique réside dans la rapidité par laquelle est établi un résultat, généralement dès la fin de l'analyse, alors qu'une attente de plusieurs jours est le plus souvent nécessaire pour obtenir les résultats de traitements réalisés sur des tissus prélevés lors d'une biopsie.

Toutefois, une analyse par voie optique du type précité présente également un inconvénient important, souvent rédhibitoire, à savoir un taux très élevé de faux diagnostics. Des faux diagnostics sont bien entendu difficilement acceptables par les patients et par le corps médical, notamment lorsqu'ils concernent des pathologies graves telles qu'un cancer par exemple.

Dans le cadre de la présente invention, on entend par taux de faux négatifs le pourcentage statistique d'indication d'un diagnostic négatif d'une pathologie donnée, alors qu'un contrôle postérieur par des méthodes usuelles telles qu'une biopsie par exemple, indique la présence réelle de cette pathologie.

On présente ci-après, à titre d'illustration, différentes techniques d'analyse optique connues, avec le taux de faux négatifs correspondants.

Une première technique d'analyse connue, qui est notamment décrite dans le brevet US-4 930 516, consiste à induire une fluorescence endogène dans le tissu vivant à l'aide d'un faisceau lumineux présentant une longueur d'onde adaptée. Comme premier exemple de diagnostic susceptible d'être effectué à partir de cette analyse optique, on peut citer le cas du cancer de l'oesophage. A cet effet, la lumière d'excitation est engendrée par un laser à colorant émettant à 410 nm à travers la fibre optique et le diagnostic est obtenu par une analyse différentielle normalisée des spectres de fluorescence endogène du tissu vivant en cause. Dans ce cas, on obtient couramment des taux de faux négatifs de plusieurs pour cent. Dans un deuxième exemple qui est relatif à l'analyse du cancer du col de l'utérus, pour laquelle l'excitation optique est engendrée par un laser à azote émettant à 337 nm et le diagnostic est effectué par l'analyse de la pente de fluorescence endogène, on obtient des taux de faux négatifs de dix pour cent. Ce dernier exemple est caractéristique d'une situation totalement inacceptable pour un médecin, pour laquelle une patiente sur dix verra son cancer s'aggraver dans l'avenir, car il n'a pu être détecté par la technique d'analyse utilisée.

Une autre technique connue consiste à analyser l'amplitude de la lumière rétrodiffusée par le tissu vivant en fonction de la longueur d'onde émise. Comme exemple de diagnostic, on peut citer dans ce cas la recherche d'un cancer du foie, pour laquelle la source lumineuse émet une lumière blanche couvrant l'ensemble du spectre visible de 300 nm à 750 nm, les fibres d'émission sont au contact du tissu suspect, les fibres de réception sont également au contact du tissu au voisinage des fibres d'émission, et le diagnostic est obtenu par l'analyse spectrale de la lumière de diffusion élastique des photons diffusés dans le tissu. Avec cette dernière technique, il est courant d'obtenir des taux de faux négatifs de trois pour cent.

Aucun de ces dispositifs ou méthodes d'analyse optique connus ne présente donc une fiabilité suffisante pour permettre au corps médical d'accorder une confiance totale à ses résultats. Ces dispositifs usuels d'analyse optique peuvent, tout au plus, servir à conforter un résultat obtenu par un autre type d'analyse beaucoup plus fiable.

La présente invention a pour objet de remédier aux inconvénients précités. Elle concerne un système d'analyse optique d'un tissu vivant, notamment du corps humain, qui est peu invasif et très fiable.

A cet effet, selon l'invention, ledit système d'analyse optique d'un tissu vivant, comportant n dispositifs d'analyse optique qui sont susceptibles de mettre en oeuvre des techniques d'analyse différentes, n étant un entier supérieur ou égal à 3, et une unité centrale, chacun desdits dispositifs d'analyse optique mettant en oeuvre, lorsqu'il est déclenché, une technique d'analyse particulière du tissu vivant de manière à pouvoir émettre une information positive ou négative sur la présence d'une pathologie particulière sur ledit tissu vivant et comprenant :
- au moins une source lumineuse susceptible d'engendrer au moins un faisceau lumineux ;
- au moins une première fibre optique susceptible de transmettre un faisceau lumineux engendré par ladite source lumineuse, sur un tissu vivant à analyser ;
- au moins une seconde fibre optique susceptible de recevoir et de transmettre un rayonnement lumineux provenant dudit tissu vivant illuminé par ledit faisceau lumineux ;
- au moins un capteur optique susceptible de mesurer un paramètre particulier dudit rayonnement lumineux transmis par ladite seconde fibre optique ; et
- un moyen de traitement pour émettre, au moins en fonction des mesures réalisées par ledit capteur optique, une information positive ou négative sur la présence d'une pathologie particulière sur ledit tissu vivant analysé,
est remarquable en ce qu'il comporte de plus :
- des moyens de sélection actionnables, pour sélectionner un nombre p desdits n dispositifs d'analyse optique différents, p étant un entier supérieur à 1 et inférieur à n ; et
- des moyens de déclenchement actionnables, pour déclencher chacun des p dispositifs d'analyse optique qui ont été sélectionnés par l'inter-médiaire desdits moyens de sélection de sorte que chacun d'eux met en oeuvre sa technique d'analyse particulière et émet une information positive ou négative sur la présence d'une pathologie,
et en ce que ladite unité centrale comprend les moyens de traitement desdits dispositifs d'analyse optique et est telle que si les moyens de traitement d'au moins q desdits p dispositifs d'analyse optique qui ont été sélectionnés et déclenchés, ont émis une information négative, q étant un entier supérieur à 1 et inférieur ou égal à p, elle émet un diagnostic négatif sur la présence de ladite pathologie sur ledit tissu vivant.

Ainsi, grâce à l'invention, ledit système présente les avantages précités (invasivité réduite, résultat rapide) des dispositifs d'analyse optique.

De plus, comme l'absence d'une pathologie est diagnostiquée uniquement si plusieurs (un nombre q de) dispositifs d'analyse optique ont détecté simultanément l'absence de cette pathologie, ledit système est particulièrement fiable. En tout cas, l'invention est réalisée, comme on le verra plus en détail ci-dessous, pour que le taux de faux diagnostics pathologiques négatifs soit inférieur au taux (généralement de l'ordre de un pour cent) requis dans la profession médicale.

Dans un mode de réalisation préféré, l'entier q est supérieur à p/2, et ladite unité centrale est, de plus, telle que si les moyens de traitement d'au moins q desdits p dispositifs d'analyse optique qui ont été sélectionnés et déclenchés, ont émis une information positive, elle émet un diagnostic positif sur la présence de ladite pathologie sur ledit tissu vivant.

Ainsi, le système conforme à l'invention est en mesure de diagnostiquer de façon fiable, non seulement l'absence d'une pathologie sur un tissu vivant, mais également sa présence.

De préférence, q est égal à p de sorte que l'absence (ou la présence) d'une pathologie est diagnostiquée si et uniquement si tous les p dispositifs d'analyse optique sélectionnés et déclenchés ont tous émis une information négative (ou une information positive) sur la présence de cette pathologie.

De façon avantageuse, toutes lesdites première et seconde fibres optiques desdits n dispositifs d'analyse optique sont agencées dans une seule et même gaine souple présentant un diamètre qui est inférieur à une valeur de diamètre maximale, de préférence deux millimètres. Ceci permet notamment d'utiliser ladite gaine dans des endoscopes médicaux usuels.

Par ailleurs, avantageusement :
- au moins l'une desdites sources lumineuses engendre un faisceau lumineux, dont le spectre optique recouvre sensiblement l'ensemble du spectre visible ; et/ou
- au moins l'une desdites sources lumineuses comporte une pluralité de lasers, dont chacun engendre un faisceau lumineux présentant une seule longueur d'onde qui est différente de celles des autres lasers.

En outre, de façon avantageuse, au moins l'un desdits capteurs optiques comporte au moins l'un des moyens suivants :
- une photodiode ;
- un photomultiplicateur ; et
- un spectrographe.

Dans un mode de réalisation particulier, lesdits moyens de sélection permettent (à un opérateur, par exemple un médecin) de choisir une pathologie particulière et ils sélectionnent automatiquement p dispositifs d'analyse optique qui sont associés au choix de cette pathologie particulière. Ainsi, pour rechercher une pathologie particulière chez un être humain, un médecin aura juste :
- à choisir (par l'intermédiaire des moyens de sélection) la pathologie qu'il soupçonne (par exemple un cancer de l'oesophage) ;
- à disposer l'extrémité distale des fibres optiques au contact de la lésion suspectée, en particulier par l'intermédiaire de moyens appropriés et connus tels qu'un endoscope par exemple ; et
- à déclencher l'analyse et le diagnostic (par l'intermédiaire desdits moyens de déclenchement).

Dans un mode de réalisation préféré, le système conforme à l'invention comporte au moins six dispositifs d'analyse optique différents, qui mettent en oeuvre des techniques d'analyse particulières, pour analyser respectivement sur le tissu vivant :
- la fluorescence, endogène ou exogène ;
- la diffusion optique élastique multispectrale ;
- l'ischémie tissulaire ;
- l'oxymétrie sanguine ;
- le spectre doppler de l'écoulement sanguin ; et
- la photopléthysmographie vasculaire.

Selon l'invention, dans ce cas, pour rechercher :
- un cancer de l'oesophage, les dispositifs d'analyse optique analysant respectivement la fluorescence, la diffusion optique élastique multispectrale et le spectre doppler de l'écoulement sanguin sont sélectionnés ;
- un cancer du poumon, les dispositifs d'analyse optique analysant respectivement la fluorescence, la diffusion optique élastique multispectrale et le spectre doppler de l'écoulement sanguin sont sélectionnés ;
- un cancer du foie, les dispositifs d'analyse optique analysant respectivement la diffusion optique élastique multispectrale, l'oxymétrie sanguine et la photopléthysmographie vasculaire sont sélectionnés ;
- un mélanome de la peau, les dispositifs d'analyse optique analysant respectivement la diffusion optique élastique multispectrale, l'oxymétrie sanguine et le spectre doppler de l'écoulement sanguin sont sélectionnés ;
- un cancer de la vessie, les dispositifs d'analyse optique analysant respectivement la fluorescence endogène, la diffusion optique élastique multispectrale et la photopléthysmographie vasculaire sont sélectionnés ;
- un infarctus du myocarde, les dispositifs d'analyse optique analysant respectivement l'ischémie tissulaire, l'oxymétrie sanguine et la photopléthysmographie vasculaire sont sélectionnés ;
- un cancer neurologique, les dispositifs d'analyse optique analysant respectivement la fluorescence, la diffusion optique élastique multispectrale et le spectre doppler de l'écoulement sanguin sont sélectionnés ;
- un cancer ORL, les dispositifs d'analyse optique analysant respectivement la diffusion optique élastique multispectrale, l'ischémie tissulaire et le spectre doppler de l'écoulement sanguin sont sélectionnés ;
- un cancer du col, les dispositifs d'analyse optique analysant respectivement la fluorescence, la diffusion optique élastique multispectrale et la photopléthysmographie sont sélectionnés ;
- une inflammation bactérienne, les dispositifs d'analyse optique analysant respectivement la fluorescence, le spectre doppler de l'écoulement sanguin et la photopléthysmographie sont sélectionnés ; et
- une brûlure, les dispositifs d'analyse optique analysant respectivement la fluorescence, l'oxymétrie sanguine et le spectre doppler de l'écoulement sanguin sont sélectionnés.

Les figures du dessin annexé feront bien comprendre comment l'invention peut être réalisée. Sur ces figures, des références identiques désignent des éléments semblables.
La figure 1 est le schéma synoptique d'un système d'analyse conforme à l'invention.
La figure 2 montre schématiquement un dispositif d'analyse optique usuel qui fait partie d'un système d'analyse conforme à l'invention.
La figure 3 montre schématiquement des dispositifs d'analyse optique qui font partie d'un mode de réalisation préféré d'un système d'analyse conforme à l'invention.
La figure 4 est une vue schématique en coupe transversale d'une gaine souple comprenant des fibres optiques de transmission de lumière.
Les figures 5 et 6 montrent schématiquement des modes de réalisation particuliers de dispositifs d'analyse optique qui font partie d'un système d'analyse conforme à l'invention.
Le système 1 conforme à l'invention et représenté schématiquement sur la figure 1 est destiné à l'analyse optique d'un tissu vivant Tv, principalement d'un être humain.

Selon l'invention, ledit système 1 comporte :
- un ensemble E de n dispositifs d'analyse optique D1 à Dn, de type usuel, n étant un entier supérieur ou égal à 3. Cet ensemble E comporte notamment un ensemble E1 de sources lumineuses, un ensemble E2 de premières fibres optiques (d'émission), un ensemble E3 de secondes fibres optiques (de réception) et un ensemble E4 de capteurs optiques, comme précisé ci-dessous. Chacun desdits dispositifs d'analyse optique D1 à Dn est susceptible de mettre en oeuvre une technique d'analyse particulière du tissu vivant Tv de manière à pouvoir émettre une information positive ou négative sur la présence d'une pathologie particulière sur ledit tissu vivant Tv ;
- des moyens de sélection 2 actionnables par un opérateur, en particulier un médecin, pour sélectionner un nombre p desdits n dispositifs d'analyse optique D1 à Dn, p étant un entier supérieur à 1 et inférieur à n ;
- des moyens de déclenchement 3 actionnables par un opérateur, pour déclencher chacun des p dispositifs d'analyse optique qui ont été sélectionnés par l'intermédiaire desdits moyens de sélection 2 de sorte que chacun d'eux met alors en oeuvre sa technique d'analyse particulière et émet une information positive ou négative sur la présence d'une pathologie particulière ; et
- une unité centrale 4 qui est telle que si au moins q desdits p dispositifs qui ont été sélectionnés et déclenchés, q étant un entier supérieur à 1 et inférieur ou égal à p, ont émis une information négative, elle émet un diagnostic négatif sur la présence de ladite pathologie sur le tissu vivant Tv analysé.

Ainsi, comme grâce à l'invention, l'absence d'une pathologie est uniquement diagnostiquée si une pluralité de q dispositifs d'analyse optique D1, D2, ..., Dn ont détecté simultanément l'absence de cette pathologie, le système 1 est particulièrement fiable. En tout cas, l'invention est réalisée, comme on le verra plus en détail ci-dessous, pour que le taux de faux diagnostics pathologiques négatifs soient inférieur au taux (un pour cent environ) requis dans la profession médicale.

Dans un mode de réalisation préféré, l'entier q est supérieur à p/2, et, si les moyens de traitement d'au moins q desdits p dispositifs d'analyse optique qui ont été sélectionnés et déclenchés, ont émis une information positive, ladite unité centrale 4 émet un diagnostic positif sur la présence de ladite pathologie sur ledit tissu vivant.

Ainsi, le système 1 conforme à l'invention est en mesure de diagnostiquer de façon fiable, non seulement l'absence d'une pathologie sur un tissu vivant Tv, mais également sa présence.

De préférence, q est égal à p de sorte que l'absence (ou la présence) d'une pathologie est diagnostiquée si et uniquement si tous les p dispositifs d'analyse optique sélectionnés et déclenchés ont émis une information négative (ou une information positive) sur la présence de cette pathologie.

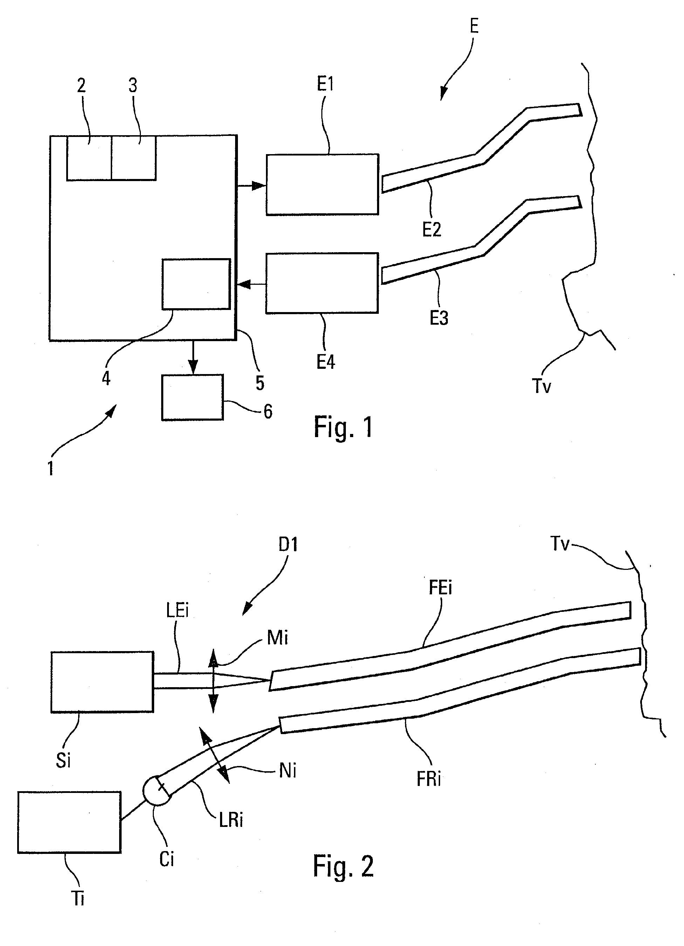

Lesdits dispositifs d'analyse optique D1 à Dn, de type usuel, présentent tous au moins les caractéristiques générales représentées à titre d'illustration pour un dispositif D1 sur la figure 2, c'est-à-dire ils comprennent tous :
- au moins une source lumineuse Si susceptible d'engendrer au moins un faisceau lumineux LEi ;
- au moins une première fibre optique FEi susceptible de transmettre sur un tissu vivant Tv un faisceau lumineux LEi engendré par ladite source lumineuse Si et reçu par l'intermédiaire d'un moyen (lentille) de focalisation Mi ;
- au moins une seconde fibre optique FRi susceptible de recevoir et de transmettre un rayonnement lumineux LRi provenant dudit tissu vivant Tv éclairé par ledit faisceau lumineux LEi ;
- au moins un capteur optique Ci qui est susceptible de mesurer un paramètre particulier dudit rayonnement lumineux LRi transmis par ladite seconde fibre optique FRi, via un moyen (lentille) de focalisation Ni ; et
- un moyen de traitement Ti pour émettre, au moins en fonction des mesures réalisées par ledit capteur optique Ci, une information positive ou négative sur la présence d'une pathologie particulière sur ledit tissu vivant Tv analysé.

Par conséquent, le faisceau lumineux LEi qui est engendré par la source lumineuse Si est amené sur le tissu vivant Tv, via la lentille de focalisation Mi et la fibre optique FEi. En pénétrant dans le tissu vivant Tv, ce faisceau lumineux LEi y induit un phénomène optique (par exemple une fluorescence qui est émise à des longueurs d'onde supérieures à celle du faisceau lumineux LEi ou une rétrodiffusion à la même longueur d'onde, dont l'analyse de l'amplitude ou de la modulation de fréquence par effet doppler permet de réaliser le diagnostic recherché) qui engendre un nouveau rayonnement lumineux, dont une partie LRi est amenée au moyen de traitement Ti, via la fibre optique FRi et la lentille de focalisation Ni, pour y être analysée. La comparaison des caractéristiques de ce rayonnement lumineux LRi induit et de celles du faisceau lumineux LEi initial permet de déduire, en fonction de techniques d'analyse particulières connues et précisées ci-dessous, la présence ou non d'une pathologie particulière.

Ainsi, grâce à l'invention, ledit système 1 présente tous les avantages des dispositifs d'analyse optique usuels, du type de celui D1 représenté sur la figure 2, à savoir notamment une invasivité réduite et des résultats obtenus rapidement.

Comme on le verra plus en détail ci-dessous :
- ladite source lumineuse Si peut comporter :
   · une source Sb (par exemple une lampe à filament de tungstène halogène) émettant une lumière blanche LEb, c'est-à-dire présentant un spectre optique très large couvrant au moins le spectre visible, mais pouvant s'étendre du proche ultraviolet (300 nm environ) au proche infrarouge (1500 nm environ) ; ou
   · une seule source laser S1 ; ou
   · une pluralité de sources laser S1 à SI émettant chacune un faisceau lumineux LE1 à LEI présentant une longueur d'onde particulière. Les sources laser SO et S1 à SI peuvent être de tout type connu, par exemple de simples diodes laser, des diodes laser doublées en fréquence ou un laser solide pompé par diodes et doublé ou triplé en fréquence ; et
- au moins l'un desdits capteurs optiques Ci peut comporter au moins l'un des moyens suivants :
   · une photodiode ;
   · un photomultiplicateur ; et
   · un spectrographe.

De préférence, les moyens 2 et 3 et l'unité centrale 4 sont intégrés dans un ordinateur 5, notamment un "ordinateur personnel". De plus, les sources lumineuses Si ont leur alimentation propre (non représentée) sous le contrôle d'un bloc de commande (non représenté) qui est lui même activé par l'ordinateur 5. Les divers signaux délivrés par les capteurs optiques Ci préalablement activés par l'ordinateur 5 sont d'abord archivés sous forme digitale, avant d'être analysés par le programme approprié situé dans l'unité centrale 4 de l'ordinateur 5. Cette dernière compare ensuite ces résultats avec les données qu'elle a en mémoire pour les pathologies considérées afin de délivrer un diagnostic. A cet effet, l'ordinateur 5 est associé à (ou comprend) un moyen 6 de visualisation du diagnostic, par exemple un écran de visualisation ou une imprimante qui fournit les résultats sur un support papier.

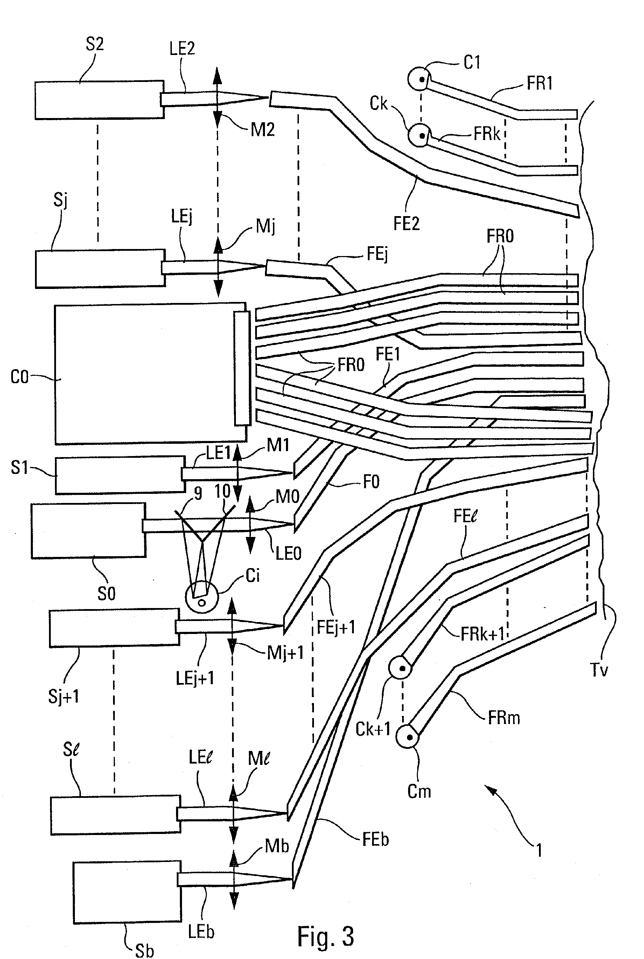

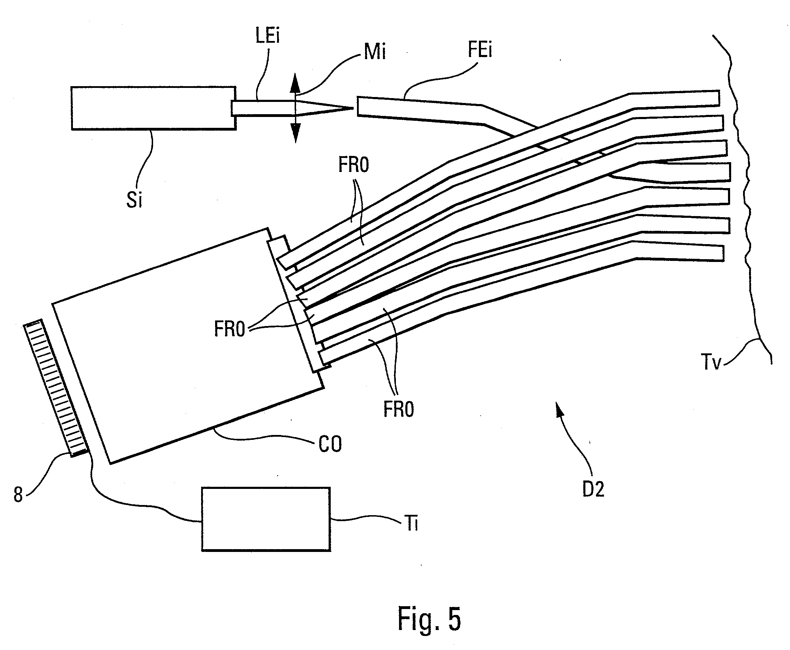

Dans le mode de réalisation préféré représenté partiellement sur la figure 3, ledit système 1 comporte :
- une source Sb de lumière blanche qui est associée à un moyen de focalisation Mb et à une fibre optique FEb ;
- des sources laser SO et S1 à Sl (S1, S2, ..., Sj, Sj+1, ..., Sl) qui sont associées respectivement à des moyens de focalisation M0 et M1 à M1 et à des fibres optiques F0 et FE1 à FEI. On notera que l'entier est compris entre 2 et (l-1) ;
- un spectrographe CO qui est associé à des fibres optiques FRO ; et
- des capteurs optiques C1 à Cm (C1, ..., Ck, Ck+1, ..., Cm) qui sont associés à des fibres optiques FR1 à FRm. On notera que l'entier k est compris entre 1 et (m-1).

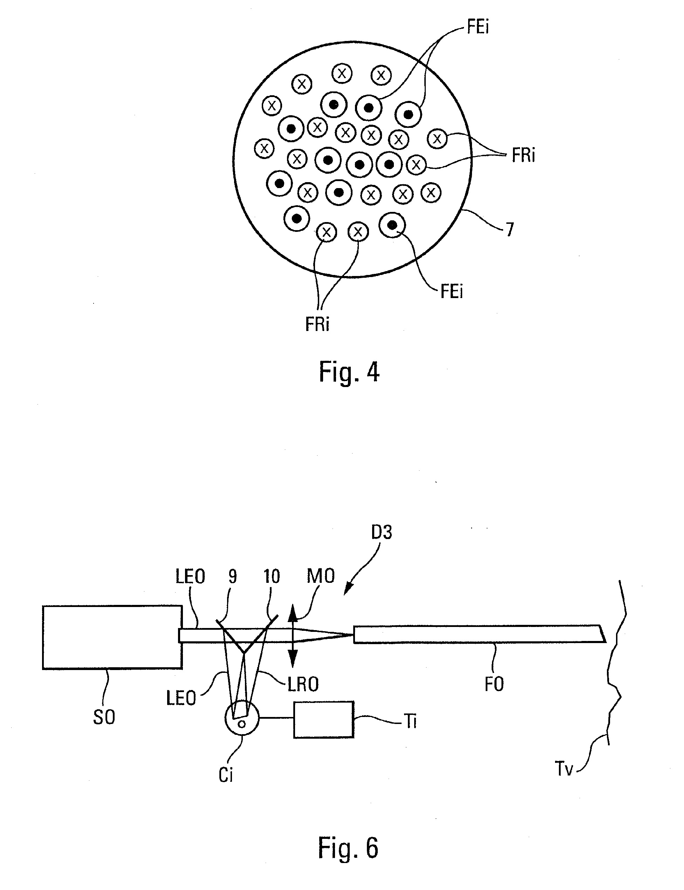

De plus, toutes les fibres optiques, c'est-à-dire aussi bien les fibres optiques d'émission FEi (FEb, F0 et FE1 à FEI) que les fibres optiques de réception FRi (FO et FR0 à FRm), sont agencées, comme montré sur la figure 4, dans une seule et même gaine souple 7 présentant un diamètre qui est inférieur à une valeur de diamètre maximale, de préférence deux millimètres. Ce diamètre est généralement requis pour pouvoir pénétrer dans le canal opératoire d'endoscopes ou de coelioscopes médicaux.

Dans un mode de réalisation particulier, lesdits moyens de sélection 2 permettent à un opérateur de choisir (par exemple manuellement ou vocalement) une pathologie particulière et ils sélectionnent automatiquement p dispositifs d'analyse optique particuliers, qui sont associés au choix de cette pathologie particulière. Ainsi, dans ce cas, pour rechercher une pathologie particulière chez un être humain, un opérateur, notamment un médecin, aura juste :
- à choisir (par l'intermédiaire des moyens de sélection 2) la pathologie qu'il soupçonne (par exemple un cancer de l'oesophage) ;
- à disposer l'extrémité distale des fibres optiques FEi et FRi (c'est-à-dire de la gaine 7) au contact de la lésion suspectée (tissu Tv), en particulier par l'intermédiaire de moyens appropriés et connus tels qu'un endoscope par exemple ; et
- à déclencher l'analyse et le diagnostic (par l'intermédiaire desdits moyens de déclenchement 3).

Dans un mode de réalisation préféré, le système 1 comporte au moins six dispositifs d'analyse optique différents et connus, qui mettent en oeuvre des techniques d'analyse particulières pour analyser, respectivement, sur le tissu vivant Tv :
- la fluorescence endogène ;
- la diffusion optique élastique multispectrale ;
- l'ischémie tissulaire ;
- l'oxymétrie sanguine ;
- le spectre doppler de l'écoulement sanguin ; et
- la photopléthysmographie vasculaire.

Dans ce cas, selon l'invention, en fonction de la pathologie recherchée, on met en oeuvre à chaque fois trois techniques d'analyse spécifiques, parmi les six techniques d'analyse particulières précitées, tel que représenté sur le tableau suivant.

| Technique Pathologie | Fluorescence | Spectroscopie de diffusion | Ischémie NaDH | Oxymétrie | Doppler | Photo-plethysmo-graphie |
|---|---|---|---|---|---|---|
| Cancer de l'oesophage | X | X | | | X | |
| Cancer du poumon | X | X | | | X | |
| Cancer du foie | | X | | X | | X |
| Mélanome de la peau | | X | | X | X | |
| Cancer de la vessie | X | X | | | | X |
| Infarctus du myocarde | | | X | X | | X |
| Cancer neurologique | X | X | | | X | |
| Cancer ORL | | X | X | | X | |
| Cancer du col | X | X | | | | X |
| Inflammation bactérienne | X | | | | X | X |
| Brûlure | X | | | X | X | |

On précise à présent les techniques d'analyse connues précitées.

La première technique d'analyse précitée peut être mise en oeuvre par un dispositif D2 représenté sur la figure 5 (et intégré dans le système 1) et consiste à analyser la fluorescence, endogène ou exogène, des tissus suspects avec un laser Si de longueur d'onde appropriée qui, pour le cas du cancer de l'oesophage par exemple, est un laser ultraviolet émettant à 350 nm. Pour cet exemple, le spectre de fluorescence est analysé entre 390 et 550 nm. Pour des maladies inflammatoires, on utilise par exemple un laser Si émettant dans le vert et on examine la fluorescence des porphyrines dans la partie rouge du spectre. Sur la figure 5, on peut voir que les fibres de réception FR0 sont beaucoup plus nombreuses (environ 10) et entourent la fibre d'émission FEi au niveau de l'extrémité distale du faisceau de fibres, car le niveau du signal de fluorescence est beaucoup plus faible qu'une rétrodiffusion à la même longueur d'onde. Ainsi, pour rechercher simultanément les deux pathologies indiquées ci-dessus, le système 1 comporte, dans un mode de réalisation particulier non représenté, à l'extrémité distale, deux fibres d'émission de lasers ultraviolet et vert, qui sont au contact l'une de l'autre et qui sont entourées par dix fibres de réception FR0. Ces deux fibres d'émission sont connectées chacune à un laser particulier alors que les dix fibres de réception FR0 sont disposées verticalement sur la fente d'entrée d'un seul et unique spectrographe CO. Le spectrographe C0 est suivi d'un détecteur linéaire 8 tel qu'une barrette de photodiodes.

La deuxième technique connue précitée consiste à envoyer sur le tissu vivant Tv tout un spectre de longueurs d'onde distinctes obtenues, soit à partir de lasers distincts couplés chacun à une fibre d'émission propre, soit à partir d'une source Sb de lumière blanche usuelle envoyée dans une seule fibre, et à recevoir le signal rétrodiffusé aux mêmes longueurs d'onde à travers les mêmes fibres optiques que les fibres de réception FR0 de la fluorescence à l'entrée d'un spectrographe CO. Toutes les fibres d'émission des lasers peuvent être disposées au voisinage des fibres de réception de la fluorescence et à l'extérieur, car le niveau de signal en rétrodiffusion est bien supérieur à celui obtenu en fluorescence. Le spectre de diffusion élastique du tissu vivant Tv apporte d'autres informations que la fluorescence, notamment les dimensions des noyaux des cellules vivantes qui rétrodiffusent la lumière. Cette information est souvent essentielle, en particulier pour les cellules cancéreuses.

La troisième technique employée est également une technique de mesure de la fluorescence, mais adaptée à la mesure d'ischémie du tissu vivant Tv considéré. L'ischémie, c'est-à-dire le manque d'oxygénation du tissu vivant Tv, est directement liée au métabolisme propre des cellules correspondantes et se traduit par une variation importante de l'intensité de la fluorescence de la molécule NaDH dans la partie violette du spectre visible vers 450 nm, pour une excitation dans l'ultraviolet proche vers 350 nm. On utilise donc les mêmes fibres optiques que celles représentées sur la figure 2, mais avec une analyse particulière du spectre au voisinage de 450 nm. Cette analyse permet d'émettre un diagnostic qui est particulièrement approprié aux tumeurs cancéreuses en cours de croissance rapide ou bien, dans un tout autre domaine, pour connaître de façon précise le taux de lésion irréversible du muscle cardiaque en cas d'infarctus du myocarde.

La quatrième technique employée concerne la mesure de diffusion élastique des photons laser, par les cellules sanguines souvent présentes dans les veines et artères capillaires des tissus analysés, pour déterminer de façon précise la quantité relative d'hémoglobine réduite par rapport à l'oxyhémoglobine. Pour réaliser cette mesure d'oxymétrie, il suffit de choisir deux longueurs d'onde telles que l'une (prise comme référence) corresponde à une longueur d'onde (par exemple 790 nm), pour laquelle l'hémo-globine réduite et l'oxyhémoglobine présentent exactement la même section efficace d'absorption, et l'autre (prise pour la mesure) correspond à une longueur d'onde (par exemple 670 nm), pour laquelle la différence d'absorption des deux molécules d'hémoglobine soit maximale. La mesure du rapport de l'intensité rétrodiffusée à ces deux longueurs d'onde permet de calculer facilement le taux d'oxymétrie des capillaires situés dans un tissu vivant Tv susceptible d'héberger une tumeur cancéreuse ou également de mesurer la rapidité de cicatrisation d'une plaie ancienne.

La cinquième technique de mesure du spectre doppler, qui peut être mise en oeuvre par un dispositif D3 représenté sur la figure 6 (et intégré dans le système 1), permet de connaître la répartition des vitesses de l'écoulement sanguin dans les divers capillaires du tissu vivant Tv analysé. Elle est basée sur l'analyse du spectre radiofréquence de battement interférentiel entre l'onde rétrodiffusée par les globules rouges en mouvement dans les capillaires et l'onde émise à l'entrée de la fibre optique F0 de mesure. Ce battement optique est facilement obtenu lorsque l'on utilise la même fibre optique F0 pour l'émission et pour la réception, grâce à deux lames séparatrices 9 et 10 superposant les deux signaux lumineux LEO et LRO sur la face d'entrée d'un capteur optique Ci (photodiode à avalanche ou photomultiplicateur).

La sixième technique, dite de photopléthysmographie, consiste à mesurer la variation de la pression dans les vaisseaux sanguins. Lorsque l'on éclaire le tissu vivant Tv par un faisceau laser lumineux présentant une longueur d'onde adaptée pour pénétrer jusqu'aux vaisseaux sanguins (soit environ 800 nm), la déformation de ces vaisseaux par la pression sanguine engendre une variation corrélative de la lumière rétrodiffusée dans le temps. On peut utiliser pour ce faire un dispositif qui est analogue à celui D1 représenté sur la figure 2.

## Revendications

1. Système d'analyse optique d'un tissu vivant (Tv), comportant n dispositifs d'analyse optique (D1, D2, D3) qui sont susceptibles de mettre en oeuvre des techniques d'analyse différentes, n étant un entier supérieur ou égal à 3, et une unité centrale (4), chacun desdits dispositifs d'analyse optique (D1, D2, D3) mettant en oeuvre, lorsqu'il est déclenché, une technique d'analyse particulière du tissu vivant (Tv) de manière à pouvoir émettre une information positive ou négative sur la présence d'une pathologie particulière sur ledit tissu vivant (Tv) et comprenant :
- au moins une source lumineuse (Si) susceptible d'engendrer au moins un faisceau lumineux (LEi) ;
- au moins une première fibre optique (FEi) susceptible de transmettre un faisceau lumineux (LEi) engendré par ladite source lumineuse (Si), sur un tissu vivant (Tv) à analyser ;
- au moins une seconde fibre optique (FRi) susceptible de recevoir et de transmettre un rayonnement lumineux (LRi) provenant dudit tissu vivant (Tv) illuminé par ledit faisceau lumineux (LEi) ;
- au moins un capteur optique (Ci) susceptible de mesurer un paramètre particulier dudit rayonnement lumineux (LRi) transmis par ladite seconde fibre optique (FRi) ; et
- un moyen de traitement (Ti) pour émettre, au moins en fonction des mesures réalisées par ledit capteur optique (Ci), une information positive ou négative sur la présence d'une pathologie particulière sur ledit tissu vivant (Tv) analysé,
**caractérisé en ce qu'**il comporte de plus :
- des moyens de sélection (2) actionnables, pour sélectionner un nombre p desdits n dispositifs d'analyse optique différents, p étant un entier supérieur à 1 et inférieur à n ; et
- des moyens de déclenchement (3) actionnables, pour déclencher chacun des p dispositifs d'analyse optique qui ont été sélectionnés par l'intermédiaire desdits moyens de sélection (2) de sorte que chacun d'eux met en oeuvre sa technique d'analyse particulière et émet une information positive ou négative sur la présence d'une pathologie,
et **en ce que** ladite unité centrale (4) comprend les moyens de traitement (Ti) desdits dispositifs d'analyse optique (D1, D2, D3) et est telle que si les moyens de traitement (Ti) d'au moins q desdits p dispositifs d'analyse optique qui ont été sélectionnés et déclenchés, ont émis une information négative, q étant un entier supérieur à 1 et inférieur ou égal à p, elle émet un diagnostic négatif sur la présence de ladite pathologie sur ledit tissu vivant (Tv).

2. Système selon la revendication 1,
**caractérisé en ce que** l'entier q est supérieur à p/2, et **en ce que** ladite unité centrale (4) est telle que si les moyens de traitement (Ti) d'au moins q desdits p dispositifs d'analyse optique qui ont été sélectionnés et déclenchés, ont émis une information positive, elle émet un diagnostic positif sur la présence de ladite pathologie sur ledit tissu vivant (Tv).

3. Système selon l'une des revendications 1 et 2,
**caractérisé en ce que** toutes lesdites première et seconde fibres optiques (FEi, FRi) desdits n dispositifs d'analyse optique (D1, D2, D3) sont agencées dans une seule et même gaine souple (7) présentant un diamètre qui est inférieur à une valeur de diamètre maximale.

4. Système selon l'une quelconque des revendications précédentes,
**caractérisé en ce que** ledit nombre q est égal audit nombre p.

5. Système selon l'une quelconque des revendications précédentes,
**caractérisé en ce qu'**au moins l'une desdites sources lumineuses (Sb) engendre un faisceau lumineux (LEb), dont le spectre optique recouvre sensiblement l'ensemble du spectre visible.

6. Système selon l'une quelconque des revendications précédentes,
**caractérisé en ce qu'**au moins l'une desdites sources lumineuses comporte une pluralité de lasers (S1 à SI) dont chacun engendre un faisceau lumineux (LE1 à LEI) présentant une seule longueur d'onde qui est différente de celles des autres lasers.

7. Système selon l'une quelconque des revendications précédentes,
**caractérisé en ce qu'**au moins l'un desdits capteurs optiques (Ci) comporte au moins l'un des moyens suivants :
- une photodiode ;
- un photomultiplicateur ; et
- un spectrographe.

8. Système selon l'une quelconque des revendications précédentes,
**caractérisé en ce qu'**il comporte au moins six dispositifs d'analyse optique (D1, D2, D3) différents, qui mettent en oeuvre des techniques d'analyse particulières, pour analyser respectivement sur un tissu vivant (Tv) :
- la fluorescence ;
- la diffusion optique élastique multispectrale ;
- l'ischémie tissulaire ;
- l'oxymétrie sanguine ;
- le spectre doppler de l'écoulement sanguin ; et
- la photopléthysmographie vasculaire.
